# EUROPÄISCHE PATENTSCHRIFT

(11) **EP 2 136 128 B1**
(45) Veröffentlichungstag und Bekanntmachung des Hinweises auf die Patenterteilung: **19.01.2011**
(21) Anmeldenummer: 08011296.4
(22) Anmeldetag: 20.06.2008
(51) Int. Cl.: F21S 8/00, F21V 23/00, A61B 19/00, F21V 23/04, F21Y 101/02, F21W 131/205

(54) **Operationsleuchte**
Operating light
Lampe chirurgicale

(43) Veröffentlichungstag der Anmeldung: 23.12.2009
(73) Patentinhaber: TRUMPF Medizin Systeme GmbH + Co. KG, 82178 Puchheim (DE)
(72) Erfinder: Marka, Rudolf, 85737 Ismaning (DE); Rosenheimer, Rouven, 85614 Kirchseeon (DE); Fritze, Dirk, 82275 Emmering (DE)
(74) Vertreter: Prüfer & Partner GbR European Patent Attorneys

(56) Entgegenhaltungen:
- EP-A- 0 299 196
- EP-A- 1 526 327
- EP-A- 1 568 934
- EP-A- 1 568 935
- WO-A-2007/086770
- DE-A1- 1 597 915
- DE-A1- 2 141 351
- US-A- 3 437 803
- US-A- 5 068 767
- US-A- 5 383 105

## Beschreibung

Die Erfindung betrifft eine Operationsleuchte mit einem in der Größe veränderlichen Leuchtfeld und einem veränderlichen Abstand zwischen dem Leuchtenkörper und dem Fokuspunkt der Lichtstrahlen, ohne dafür mechanische Verstellmittel aufzuweisen.

Eine Möglichkeit der Ausführung von Operationsleuchten sind so genannte Großspiegelleuchten. Hierbei ist die Lichtquelle eine Halogenlampe oder Gasentladungslampe, die im Brennpunkt eines großen Reflektors mit einem Durchmesser von ca. 500 mm bis 1000 mm angeordnet ist. Durch eine Verschiebung der Lichtquelle auf der Mittelachse des Reflektors, und damit mehr oder weniger aus dem oder in den optimalen Brennpunkt des Reflektors, wird der Durchmesser des Leuchtfelds, d.h. der beleuchtete Durchmesser im Operationsfeld vergrößert oder verkleinert und der Fokuspunkt verändert, d.h. der Abstand der hellsten beleuchteten Stelle, in der sich die reflektierten Lichtstrahlen schneiden, von dem Leuchtenkörper der Operationsleuchte auf einer Mittelachse des Leuchtenkörpers verändert. Dabei ist eine aufwändige Mechanik erforderlich, die eine prozesssichere leichtgängige Bedienung ohne großen Betätigungsaufwand des Bedieners, ermöglicht.

Eine andere Ausführung sind aufgelöste Lichtsysteme. Hierbei umfasst die Operationsleuchte in der Regel einen zentralen Scheinwerfer oder ein zentrales Lichtmodul, der/das starr am Leuchtenkörper befestigt ist und mehrere Scheinwerfer oder Lichtmodule in einer ringförmigen Anordnung um den zentralen Scheinwerfer oder das zentrale Lichtmodul aufweist. Die Veränderung der Richtung des Lichtaustritts aus den äußeren Scheinwerfern oder Lichtmodulen ist über radial verschwenkbare Leuchtmittel oder Reflektoren realisiert, oder die gesamten Scheinwerfer oder Lichtmodule sind radial schwenkbar verstellbar, so dass der Fokuspunkt der Lichtstrahlen aus den äußeren Scheinwerfern oder Lichtmodulen seinen Abstand vom Leuchtenköper auf der Mittelachse verändert. Dabei ist ebenfalls eine aufwändige Mechanik zur synchronen, prozesssicheren Verstellung der äußeren Leuchtmittel, Reflektoren oder Lichtmodulen erforderlich.

Eine weitere Art von Operationsleuchten ist ohne die Verstellmöglichkeit des Leuchtfelddurchmessers und des Abstands des Fokuspunkts ausgeführt. Hierbei sind die lichttechnischen Daten, Leuchtfelddurchmesser und Fokuspunkt für einen Arbeitspunkt optimal eingestellt. Eine Veränderung des Leuchtfelddurchmessers ist hier nur durch eine Veränderung des Abstands des Leuchtenkörpers vom Operationsfeld möglich. Die Veränderung des Abstands des Fokuspunktes ist nicht möglich. Bei aufgelösten Lichtsystemen besteht sogar die Gefahr, dass das Leuchtfeld nicht mehr homogen wirkt, sondern sich, bei geringem Abstand des Leuchtenkörpers von der Operationsstelle einzelne Lichtpunkte in der Operationsstelle abbilden.

Die Patentanmeldung EP-A-1 938 768 zeigt eine Operationsleuchte mit einer Gruppe von Haupt-Leuchtmitteln, die jeweils einen gebündelten Lichtstrahl aufweisen, die sich auf einem Punkt im Zentrum des Leuchtfelds schneiden, der einen Meter vom Leuchtenkörper entfernt ist, und eine weitere Gruppe von Zusatz-Leuchtmitteln, die zwischen den Haupt-Leuchtmitteln angeordnet sind und deren Lichtstrahlen Achsen aufweisen, die einen konzentrischen ringförmigen Lichtstrahl mit einem Durchmesser von 100 bis 200mm um den Lichtstrahl der Haupt-Leuchtmittel bilden. Die Leuchtmittel beider Gruppen sind jeweils mit einer Steuerung verbunden.

Es ist Aufgabe der Erfindung, eine Operationsleuchte zur Verfügung zu stellen, die kostengünstig die Möglichkeit bietet, den Leuchtfelddurchmesser und den Abstand des Fokuspunkts vom Leuchtenkörper zu verändern.

Die Aufgabe wird mit den Merkmalen des Anspruchs 1 gelöst. Weiterbildungen der Erfindung sind in den Unteransprüchen beschrieben.

Die Operationsleuchte bietet durch eine spezielle Anordnung und Ansteuerung der Leuchtmittel die Möglichkeit, den Leuchtfelddurchmesser und den Abstand des Fokuspunkts vom Leuchtenkörper zu verändern. Dies erfolgt ohne mechanische Verstellmittel.

Die Erfindung wird anhand eines Ausführungsbeispiels unter Bezugnahme auf die beigefügten Zeichnungen erläutert.
- Fig. 1: ist eine perspektivische Ansicht eines Ausführungsbei- spiels der erfindungsgemäßen Operationsleuchte.
- Fig. 2: ist eine Schnittansicht eines Leuchtenkörpers mit dem Verlauf verschiedener Lichtstrahlen.
- Fig. 3: zeigt eine Schnittansicht eines Leuchtenkörpers mit der Anordnung der Leuchtmittel.

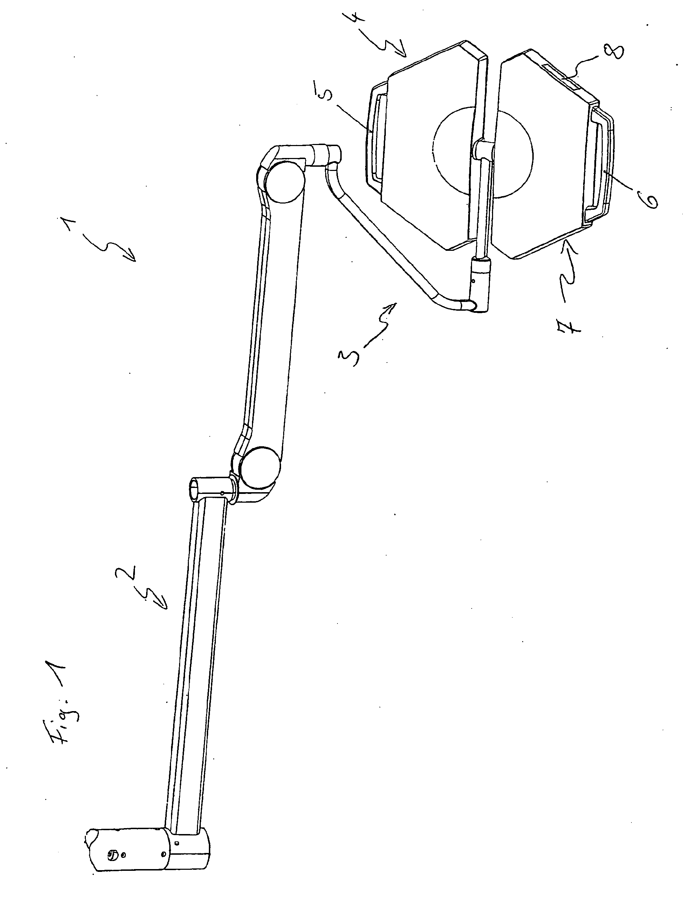

Fig. 1 zeigt eine perspektivische Ansicht eines Ausführungsbeispiels der erfindungsgemäßen Operationsleuchte 1. Die Operationsleuchte 1 umfasst ein Tragsystem 2, eine Aufhängevorrichtung 3 und einen Leuchtenkörper 4. Das Tragsystem 2 wird an einer Raumdecke, einer Wand oder einem fahrbaren Ständer befestigt. Durch das Tragsystem 2 und die Aufhängevorrichtung 3 ist der Leuchtenkörper 4 innerhalb des Aktionsradius in jeder beliebigen räumlichen Stellung und Orientierung positionierbar. Auf der nicht gezeigten, gegenüberliegenden Seite des Leuchtenkörpers 4 ist auf nahezu der gesamten Fläche eine Lichtaustrittsöffnung angeordnet, die im Betrieb auf ein Operationsfeld, das sich in einem bestimmten Abstand befindet, gerichtet ist.

Zum unsterilen Positionieren des Leuchtkörpers 4 sind an den beiden Hälften des Leuchtenkörpers 4 je ein Griff 5, 6 angebracht. Die beiden Hälften des Leuchtenkörpers 4 sind drehfest miteinander verbunden, so dass sich beim Verschwenken der einen Hälfte die andere Hälfte mit bewegt, um die Lichtaustrittsflächen immer in einer Ebene zu halten.

Innerhalb des Leuchtenkörpers 4 ist eine Steuerungsvorrichtung 7 angeordnet. Die Steuerungsvorrichtung 7 muss nicht zwingend in dem Leuchtenkörper 4 angeordnet sein, sondern kann auch in einem separaten Gehäuse, das am Leuchtenkörper 4 oder an der Aufhängevorrichtung 3 angeordnet ist, untergebracht sein. Alternativ besteht auch die Möglichkeit, dass sich die Steuerungsvorrichtung 7 in einer nicht gezeigten externen Bedieneinheit befindet, die sich in einer Medizinischen Versorgungseinheit oder in/an einer Wand befindet.

An der Außenseite des Leuchtenkörpers 4 ist eine Bedienvorrichtung 8 angeordnet. Die Bedienvorrichtung 8 kann sich aber auch in einem separaten Gehäuse befinden, das sich beispielsweise am Leuchtenkörper 4, an der Aufhängevorrichtung 3 oder in einer Medizinischen Versorgungseinheit oder in/an einer Wand befindet.

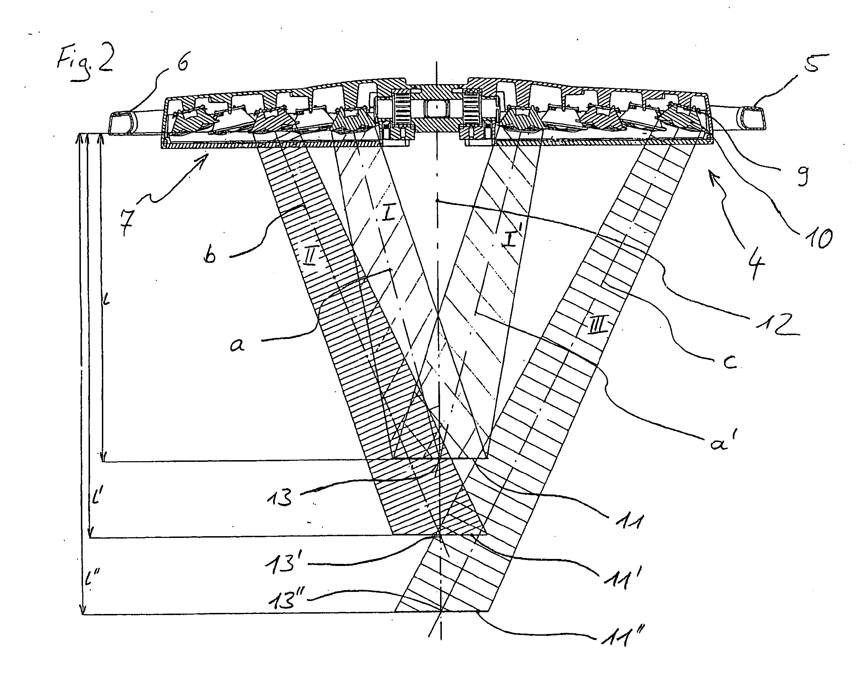

Fig. 2 ist eine Schnittansicht eines Leuchtenkörpers 4, in dem eine Mehrzahl von Leuchtmitteln 9 vorgesehen ist. Der Leuchtenkörper 4 hat eine Mittelachse 12, die senkrecht auf einer Ebene steht, in der die Leuchtmittel 9 angeordnet sind. Die Leuchtmittel 9 sind ringförmig oder entsprechend der Form des Leuchtenkörpers 4 angeordnet.

Jedes Leuchtmittel 9 ist mit einer Vorrichtung zum Bündeln des Lichts, hier einem Refraktor 10, ausgestattet. Anstelle der separaten Refraktoren 10 können auch separate Reflektoren oder Leuchtmittel mit integrierten Mitteln zum Bündeln des Lichts verwendet werden.

Das gebündelte Licht der Leuchtmittel 9, tritt jeweils in einem Lichtstrahlbündel I, I', II, III aus dem Refraktor 10 aus, das jeweils eine Achse a, a', b, c besitzt. Ein Lichtstrahlbündel I, I', II, III beleuchtet jeweils ein gesamtes Leuchtfeld 11, 11', 11".

Die Leuchtmittel 9 mit den Refraktoren 10 sind geneigt angeordnet, so dass die Achsen a, a', b, c der Lichtstrahlbündel I, I', II, III die Mittelachse 12 jeweils in einem Schnittpunkt 13, 13', 13" schneiden.

Die Erzeugung des Leuchtfelds 11 wird exemplarisch beschrieben, die Erzeugung der weiteren Leuchtfelder 11', 11" erfolgt analog.

Die Lichtstrahlbündel I, I' bilden in einer Ebene, die in einem Abstand 1, senkrecht zur Mittelachse 12 liegt, je ein Leuchtfeld. Der Schnittpunkt 13 ist der Schnittpunkt beider Achsen I, I' mit der Mittelachse 12, also ein Fokuspunkt, so dass die beiden Leuchtfelder zur Deckung kommen und gemeinsam das Leuchtfeld 11 bilden. Das Leuchtfeld 11 besitzt einen bestimmten Durchmesser D und eine definierte Verteilung der Leuchtstärke über den Durchmesser des Leuchtfelds, die normativ vorgegeben ist.

In Fig. 2 sind nur zwei Leuchtmittel 9 gezeigt, die das Leuchtfeld 11 bilden. Das Leuchtfeld 11 wird aber durch eine Schar von Lichtstrahlbündel I, I' gebildet, die von den Leuchtmittel 9 ausgestrahlt werden, die gleichmäßig über die Lichtaustrittsfläche verteilt sind und deren Achsen a, a' der Lichtstrahlbündel I, I' sich im Schnittpunkt 13 schneiden.

Die Leuchtmittel 9 sind in der Ebene gleichmäßig verteilt, um ein Hindernis, das zwischen den Leuchtenkörper 4 und das Operationsfeld eingebracht wird und dadurch ein Lichtstrahlenbündel unterbricht, was eine Schattenbildung zur Folge hat, zu unterleuchten und somit die Schattenbildung zu verhindern oder abzuschwächen.

Die Lichtstrahlbündel II, III zur Bildung der Leuchtfelder 11', 11" sind in Fig. 2 jeweils ausgehend von einem Leuchtmittel 9 dargestellt, sind jedoch im vorliegendem Ausführungsbeispiel ebenfalls jeweils eine Schar von Lichtstrahlbündel, die sich in den Schnittpunkten 13', 13" schneiden und Fokuspunkte in den Abständen l', l" bilden.

Aufgrund des in der relevanten Norm festgelegten Abstands des Leuchtenkörpers 4 von der Operationsstelle von 100 cm, haben sich in empirischen Versuchen jeweils die Abstände l = ca. 90 cm, l' = ca. 100 cm und l" = ca. 110 cm als günstig erwiesen. Sie können als festgelegte Abstände definiert werden. Die Abstände l, l', l" entsprechen dem jeweiligen Abstand des Leuchtenkörpers 4 vom Operationsfeld, auf dem das Leuchtfeld 11, 11', 11" abgebildet wird. In Abhängigkeit von der Größe des Leuchtenkörpers 4 können die Werte unterschiedlich sein.

Bei der Verwendung von mehreren Leuchtmitteln 9 die auf jeweils ein Leuchtfeld 11, 11', 11" gerichtet sind, können verschiedenfarbige Leuchtmittel 9 verwendet werden. Dabei besteht die Möglichkeit, die resultierende Farbtemperatur des Lichts in einem bestimmten Farbtemperaturbereich einzustellen.

Die Leuchtmittel 9 werden in diesem Ausführungsbeispiel durch LEDs gebildet, können aber auch als Halogenlampen oder Gasentladungsleuchten, ggf. mit Farbfiltern ausgebildet sein.

In dem Leuchtenkörper 4 ist die nicht gezeigte Steuerungsvorrichtung 7 angeordnet. Die Steuerung weist Mittel zum Dimmen und Ein- und Ausschalten der Leuchtmittel 9, wie z.B. Stromregler, Mittel zum Übertragen von Schalt- und Einstellinformationen der Schalt- und Einstellelemente der Bedienvorrichtung 8, einen Speicherbereich zum Abspeichern von Betriebsparametern und eine CPU, die aus den Schalt- und Einstellinformationen, an Hand der abgespeicherten Betriebsparameter, die erforderlichen Einstellungen für die Mittel zum Dimmen und Ein- und Ausschalten der Leuchtmittel berechnet oder bestimmt, auf.

Die Steuerungsvorrichtung 7 ist mit den Leuchtmitteln 9 verbunden, die gruppenweise angesteuert werden. Eine Gruppe wird aus mehreren Leuchtmitteln 9 gebildet, die mit den gleichen Leistungsparametern angesteuert werden. Die einzelnen Gruppen bestehen jeweils nur aus Leuchtmitteln 9, deren Lichtstrahlen I, I', II, II den gleichen Schnittpunkt 13, 13', 13" mit der Mittelachse 12 haben. Es sind aber mehrere Gruppen möglich, deren Lichtstrahlen I, I', II, III der Leuchtmittel 9 auf den gleichen Schnittpunkt 13, 13', 13" gerichtet sind.

Die Steuerungsvorrichtung 7 ist ebenfalls mit der Bedienvorrichtung 8 verbunden. An der Bedienvorrichtung 8 ist
- ein Element zum Ein-/Ausschalten,
- ein Element zur Abstandseinstellung und
- ein Element zur Helligkeitseinstellung
   vorgesehen.

Das Element zum Ein-/Ausschalten schaltet die Operationsleuchte 1 von einem Standby-Modus, in der die Leuchtmittel 9 nicht leuchten, in einen Betriebsmodus. Dabei werden die Leuchtmittel 9 entsprechend der Einstellung der Einstellelemente betrieben.

Zum vollständigen Ausschalten durch Abschalten der Stromversorgung ist ein nicht gezeigter externer Hauptschalter vorgesehen.

Das Element zur Abstandseinstellung gibt an die Steuerungsvorrichtung 7 die Information, auf welchen Abstand des Leuchtfelds 11, 11', 11" vom Leuchtenkörper 4 die Operationsleuchte eingestellt werden soll. Das Element zur Abstandseinstellung kann in dieser Ausführungsform auf die drei Abstände l, l' und l" eingestellt werden. In einer alternativen Ausführungsform ist auch ein Element zur Abstandseinstellung von weiteren diskreten Abstandswerten oder einer stufenlosen Abstandseinstellung möglich.

Im Betrieb bei einem eingestellten Abstand, der einem der festgelegten Abstände l, l', l" entspricht, auf die die Leuchtmittel mit den Lichtstrahlen I, I', II, III gerichtet sind, werden im vorliegenden Ausführungsbeispiel durch die Steuerungsvorrichtung 7 die Leuchtmittel 9 mit einer größeren Leistung angesteuert, deren Achse a, a', b, c der Lichtstrahlen I, I', II, III auf den jeweiligen Schnittpunkt 13, 13', 13" gerichtet ist. So werden beispielsweise bei einem eingestellten Abstand von 90 cm die Leuchtmittel mit den Lichtstrahlen I, I' mit einer größeren Leistung angesteuert, deren Achsen a, a' sich im Schnittpunkt 13 im Abstand 1 mit der Mittelachse 12 schneiden und das Leuchtfeld 11 im Operationsfeld bilden. Analog werden bei einem Abstand von 100 cm die Leuchtmittel 9 mit einer größeren Leistung angesteuert, die die Lichtstrahlen II erzeugen, deren Achsen b sich im Schnittpunkt 13' mit der Mittelachse 12 schneiden und das Leuchtfeld 11' im Operationsfeld bilden, und bei einem Abstand von 110 cm werden die Leuchtmittel 9 betrieben, die die Lichtstrahlen III erzeugen, deren Achsen c sich im Schnittpunkt 13" mit der Mittelachse 12 schneiden und das Leuchtfeld 11" im Operationsfeld bilden. Mit größerer Leistung angesteuert heißt, dass bei einer maximalen Helligkeitseinstellung der Operationsleuchte 1, die jeweiligen Leuchtmittel mit maximaler Leistung betrieben werden. Zur Erreichung der vorgegebenen Leuchtstärke werden ergänzend die Leuchtmittel 9, deren Lichtstrahlen auf benachbarte Schnittpunkte 13, 13', 13" gerichtet sind, mit einer bestimmten Leistung betrieben. Die jeweiligen Leistungseinstellungen werden empirisch ermittelt und in der Steuerungsvorrichtung 7 abgespeichert. Bei einer geringeren Helligkeitseinstellung ändern sich die Leistungswerte anteilig.

In der alternativen Ausführungsform werden ausschließlich die Leuchtmittel 9 betrieben, deren Lichtstrahlbündel I, I', II, III auf den eingestellten Abstand, der einem der festgelegten Abstände l, l', l" entspricht, gerichtet sind.

Bei einem eingestelltem Abstand, der nicht einem der festgelegten Abstände l, l', l" entspricht, werden in der alternativen Ausführungsform durch die Steuerungsvorrichtung 7 die Leuchtmittel 9, bei denen der Schnittpunkt 13, 13', 13" der Achsen a, a', b, c ihrer Lichtstrahlen I, I', II, III an den eingestellten Abstand angrenzt, betrieben. Die Helligkeitseinstellung der einzelnen Leuchtmittel 9 wird dann so gewählt, dass der Abstand des Schnittpunkts der Achsenschar, der aus den einzelnen Lichtstrahlen I, I', II, III resultierenden Achsen, mit der größten Helligkeit, mit der Mittelachse 12 des Leuchtenkörpers 4, dem eingestellten Abstand entspricht. Liegt der eingestellte Abstand beispielsweise zwischen den Abständen l' und l", werden die Leuchtmittel 9, die die Lichtstrahlen II und III erzeugen, betrieben. Die Lichtstrahlen II und III erzeugen einen resultierenden Lichtstrahl mit einer größeren Helligkeit als die der einzelnen Lichtstrahlen II und III, dessen Schnittpunkt seiner Achse mit der Mittelachse 12 zwischen den Schnittpunkten 13' und 13" der Achsen b und c mit der Mittelachse 12 liegt. Die Helligkeit der Leuchtmittel 9 für die Lichtstrahlen II und III ist so abgestimmt, dass die Achse des resultierenden Lichtstrahls die Mittelachse 12 im eingestellten Abstand schneidet. Liegt der eingestellte Abstand näher bei l" werden die Leuchtmittel 9 für die Lichtstrahlen III heller und die die Leuchtmittel 9 für die Lichtstrahlen II dunkler eingestellt, liegt der eingestellte Abstand näher bei l' werden die Leuchtmittel 9 für die Lichtstrahlen II heller und die die Leuchtmittel 9 für die Lichtstrahlen III dunkler eingestellt.

Bei diesen Einstellungen, in der der eingestellte Abstand dem tatsächlichen Abstand entspricht, erfüllt das Leuchtfeld die normativen Vorgaben bezüglich der Helligkeitsverteilung im Leuchtfeld. Durch eine Einstellung, bei der der eingestellte Abstand nicht dem tatsächlichen Abstand entspricht, verändert sich auch die Helligkeitsverteilung im Leuchtfeld. Dadurch bietet die Operationsleuchte 1 die Möglichkeit, die Helligkeitsverteilung im Leuchtfeld auf die Anforderungen des Operateurs einzustellen.

Bei einer Verstärkung der Lichtstrahlen III, also einer Einstellung des Abstands l" am Element zur Abstandseinstellung, wird das Leuchtfeld größer, sofern der tatsächliche Abstand geringer als l" ist, und die Helligkeit im Zentrum des Leuchtfelds nimmt ab. Ebenso vergrößert sich bei einer Verstärkung der Lichtstrahlen I, also einer Einstellung des Abstands 1 am Element zur Abstandseinstellung, das Leuchtfeld und die Helligkeit im Zentrum des Leuchtfelds nimmt ab, sofern der tatsächliche Abstand größer als 1 ist.

Das Element zur Helligkeitseinstellung gibt an die Steuerungsvorrichtung 7 die Einstellinformation über die eingestellte Gesamthelligkeit der Operationsleuchte 1. Die Mittel zum Dimmen und Ein- und Ausschalten werden von der Steuerungsvorrichtung 7 dann so gesteuert, dass die Verteilung der Helligkeit der einzelnen Leuchtmittel 9 unverändert bleibt und nur die Gesamthelligkeit verändert wird.

Die Betriebsparameter für die Einstellungen werden empirisch ermittelt und im Speicherbereich der Steuerungsvorrichtung 7 abgespeichert.

In einer alternativen Ausführungsform umfasst die Operationsleuchte 1 einen Abstandssensor zum Messen des Abstands zwischen dem Leuchtenkörper 4 und der Operationsstelle und Mittel zur Weitergabe des Abstands an die Steuerungsvorrichtung 7. Durch die Erfassung des Abstands des Leuchtenkörpers 4 von der Operationsstelle und Weitergabe der Abstandsinformation an die Steuerungsvorrichtung 7 ist die Steuerungsvorrichtung 7 in der Lage, den Punkt mit der maximalen resultierenden Helligkeit in dem Abstand des Leuchtenkörpers 7 von der Operationsstelle einzustellen, so dass die Operationsstelle mit der größten Helligkeit beleuchtet wird.

In einer weiteren alternativen Ausführungsform umfasst die Operationsleuchte 1 einen Helligkeitssensor, der die Helligkeit in der Operationsstelle misst und Mittel zur Weitergabe der Helligkeitsinformation an die Steuerungsvorrichtung 7. Durch die Erfassung der Helligkeit, wobei eine Möglichkeit ist, die Helligkeit im Zentrum des Leuchtfelds zu erfassen, und eine andere Möglichkeit, die durchschnittliche Helligkeit im gesamten Leuchtfeld zu erfassen, und Weitergabe der Helligkeitsinformation an die Steuerungsvorrichtung 7, ist die Steuerungsvorrichtung 7 in der Lage, den Punkt mit der maximalen resultierenden Helligkeit in dem Abstand des Leuchtenkörpers 4 so einzustellen, dass die Operationsstelle bei einer Veränderung des Abstands des Leuchtenkörpers 4 von der Operationsstelle mit der gleichen Helligkeit in dem jeweiligen Erfassungsraum beleuchtet wird. Zusätzlich umfasst in einer dieser alternativen Ausführungsformen die Operationsleuchte 1 optional einen Auslösemechanismus und Mittel zur Weitergabe einer Auslöseinformation an die Steuerungsvorrichtung 7. Die ständige Anpassung des Abstands des Punkts mit der maximalen resultierenden Helligkeit führt zu Irritationen des Operateurs, da bei einem Einführen eines Hindernisses, z.B. einem Instrument, den Händen oder des Kopfes des Operateurs, in den Lichtstrahl I, I', II, III auf Grund des sich ändernden Abstands oder der sich ändernden Helligkeit, der Abstand des Punktes nachreguliert wird. Der Auslösemechanismus erlaubt die Anpassung des Abstands des Punktes mit der maximalen resultierenden Helligkeit zu den vom Operateur gewünschten Zeitpunkten.

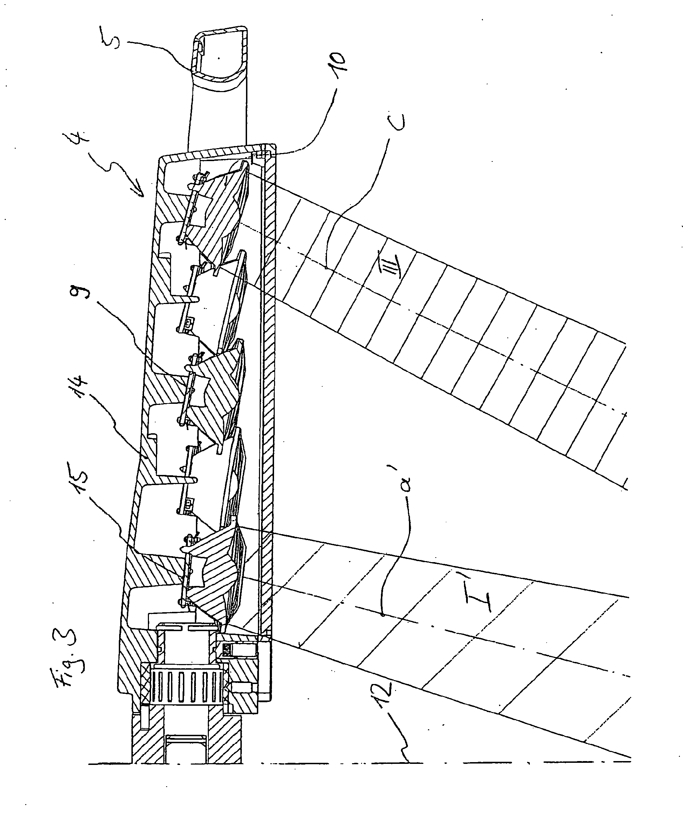

Fig. 3 zeigt eine Schnittansicht eines Leuchtenkörpers 4 mit der Anordnung der Leuchtmittel 9. Der Leuchtenkörper 4 weist ein Gehäuse 14 aus Leichtmetall oder einem geeigneten Kunststoffmaterial auf, in dem jeweils eine Befestigungsfläche 15 für jedes Leuchtmittel 9 vorgesehen ist.

Die Befestigungsflächen 15 sind so vorgesehen, dass die Leuchtmittel 9 in einer Ebene senkrecht zur Mittelachse 12 des Leuchtenkörpers 4 angeordnet sind. Damit wird eine flache Bauform erreicht, die strömungstechnisch günstig ist und somit den laminaren Luftstrom einer Turbulenzarmen Verdrängungsströmung (TAV) über dem Operationsfeld kaum beeinflusst.

Die Befestigungsflächen 15 sind gegenüber der Mittelachse 12 geneigt, so dass eine senkrechte Achse darauf parallel zu der jeweiligen Achse a, a', b, c ist und die Achse 12 in den Punkten 13, 13' und 13" schneidet. Dadurch können die identischen Leuchtmittel 9 mit Refraktoren 10 an allen Befestigungsflächen 15 verwendet werden und nur minimale Unterschiede in den Durchmessern bei den verschiedenen Abständen entstehen.

## Patentansprüche

1. Operationsleuchte (1), umfassend einen Leuchtenkörper (4) mit einer Mittelachse (12) mit mindestens zwei Leuchtmitteln (9) mit gebündelten Lichtstrahlen (I, I', II, III), wobei die gebündelten Lichtstrahlen (I, I', II, III) jeweils eine Achse (a, a', b, c) aufweisen und die Achsen (a, a', b, c)
jeweils auf die Mittelachse (12) gerichtet sind und mit der Mittelachse (12) einen Schnittpunkt (13, 13', 13") in jeweils einem Abstand (l, l', l") vom Leuchtenkörper (4) bilden, **dadurch gekennzeichnet, dass**
der Abstand (l, l', l")
von mindestens zwei Schnittpunkten (13, 13', 13") vom Leuchtenkörper (4) in Richtung der Mittelachse (12) unterschiedlich ist.

2. Operationsleuchte gemäß Anspruch 1, **dadurch gekennzeichnet, dass** Mittel zum unabhängigen Dimmen und Ein- und Ausschalten der einzelnen Leuchtmittel (9) vorhanden sind.

3. Operationsleuchte gemäß einem der vorangehenden Ansprüche, **dadurch gekennzeichnet, dass** die Leuchtmittel (9) in einer Ebene, senkrecht zur Mittelachse (12) angeordnet sind.

4. Operationsleuchte gemäß einem der vorangehenden Ansprüche, **dadurch gekennzeichnet, dass** mehrere Leuchtmittel (9) vorhanden sind, die zu Gruppen zusammengefasst den jeweiligen Punkt (13, 13', 13") beleuchten.

5. Operationsleuchte gemäß Anspruch 4, **dadurch gekennzeichnet, dass** die Leuchtmittel (9) einer Gruppe gleichmäßig über den Leuchtenkörper (4) verteilt sind.

6. Operationsleuchte gemäß einem der vorangehenden Ansprüche, **dadurch gekennzeichnet, dass** die Leuchtmittel (9) auf geneigten Befestigungsflächen (15) angebracht sind.

7. Operationsleuchte gemäß Anspruch 6, **dadurch gekennzeichnet, dass** sich senkrechte Achsen auf den jeweiligen geneigten Flächen (15) für eine Gruppe in dem Punkt (13, 13', 13") auf der Mittelachse schneiden.

8. Operationsleuchte gemäß einem der vorangehenden Ansprüche, **dadurch gekennzeichnet, dass** die Leuchtmittel (9) LEDs sind.

9. Operationsleuchte gemäß einem der vorangehenden Ansprüche, **dadurch gekennzeichnet, dass** die Leuchtmittel (9) LEDs mit Refraktoren sind.

10. Operationsleuchte gemäß einem Ansprüche 1 bis 7, **dadurch gekennzeichnet, dass** die Leuchtmittel (9) LEDs mit Reflektoren sind.

11. Operationsleuchte gemäß Anspruch 2 bis 10, **dadurch gekennzeichnet, dass** eine Steuerungsvorrichtung (7) vorgesehen ist, die die Leuchtmittel (9) bzw. die Leuchtmittelgruppen so dimmt, dass eine aus den einzelnen Lichtstrahlen (I, I', II, III) resultierende Achsenschar mit der größten Helligkeit einen Schnittpunkt mit der Mittelachse (12) hat.

12. Operationsleuchte gemäß Anspruch 11, **dadurch gekennzeichnet, dass** die Operationsleuchte (1) einen Abstandssensor zum Messen eines Abstands zwischen dem Leuchtenkörper (4) und dem Operationsstelle und Mittel zur Weitergabe der Abstandsinformation an die Steuerungsvorrichtung (7) umfasst.

13. Operationsleuchte gemäß Anspruch 11, **dadurch gekennzeichnet, dass** die Operationsleuchte (1) einen Helligkeitssensor zum Messen der Helligkeit in der Operationsstelle und Mittel zur Weitergabe der Helligkeitsinformation an die Steuerungsvorrichtung (7) umfasst.

14. Operationsleuchte gemäß einem der Ansprüche 12 oder 13, **dadurch gekennzeichnet, dass** die Operationsleuchte (1) einen Auslösemechanismus und Mittel zur Weitergabe der Auslöseinformation an die Steuerungsvorrichtung (7) umfasst.

## Claims

1. Surgical lamp (1), comprising
a lamp body (4) having a central axis (12), the lamp body comprising at least two illuminants (9) with bundled light beams (I, I', II, III), wherein the bundled light beams (I, I', II, III) comprise an axis (a, a', b, c), respectively, and the axes (a, a', b, c) are directed to the central axis (12), respectively, and the axes (a, a', b, c) form an intersection point (13, 13', 13") with the central axis (12) at a distance (l, l', l") from the light head (4), respectively, **characterized in that** the distance (l, l', l") between at least two intersection points (13, 13', 13") and the lamp body (4) in the direction of the central axis (12) is different.

2. Surgical lamp according to claim 1, **characterized in that**
means for independent dimming and switching on and switching off of the several illuminants (9) are provided.

3. Surgical lamp according to any of the preceding claims, **characterized in that**
the illuminants (9) are arranged in a plane which is perpendicular to the central axis (12).

4. Surgical lamp according to any of the preceding claims, **characterized in that**
several illuminants (9) are provided, which are collected to groups and illuminate the respective point (13, 13', 13").

5. Surgical lamp according to claim 4, **characterized in that**
the illuminants (9) of one group are evenly distributed across the lamp body (4).

6. Surgical lamp according to any of preceding claims, **characterized in that**
the illuminants (9) are attached to inclined fixing faces (15).

7. Surgical lamp according to claim 6, **characterized in that**
perpendicular axes on the respective inclined faces (15) of one group intersect in the point (13, 13', 13") on the central axis.

8. Surgical lamp according to any of preceding claims, **characterized in that**
the illuminants (9) are constituted by LEDs.

9. Surgical lamp according to any of preceding claims, **characterized in that**
the illuminants (9) are constituted by LEDs with refractors.

10. Surgical lamp according to any of claims 1 to 7, **characterized in that**
the illuminants (9) are constituted by LEDs with reflectors.

11. Surgical lamp according to any of claims 2 to 10, **characterized in that**
a control device (7) is provided which dims the illuminants (9) or the groups of illuminants in such way that a sheaf of axes which results of the several light beams (I, I', II, III), having the greatest brightness, has an intersection point with central axis (12).

12. Surgical lamp according to claim 11, **characterized in that**
the surgical lamp (1) comprises a distance sensor for measuring a distance between the lamp body (4) and the operation site and means for transmitting the distance information to the control device (7).

13. Surgical lamp according to claim 11, **characterized in that**
the surgical lamp (1) comprises a brightness sensor for measuring the brightness in the operation site and means for transmitting the brightness information to the control device (7).

14. Surgical lamp according to any of claims 12 or 13, **characterized in that**
the surgical lamp (1) comprises an actuator and means for transmitting the actuation information to the control device (7).

## Revendications

1. Luminaire chirurgical (1), comprenant un corps de luminaire (4) avec un axe médian (12) avec au moins deux moyens d'éclairage (9) ayant des rayons lumineux (I, I', II, III) focalisés, les rayons lumineux (I, I', II, III) focalisés présentant chacun un axe (a, a', b, c) et les axes (a, a', b, c) étant chacun dirigés sur l'axe médian (12) et formant, avec l'axe médian (12), un point d'intersection (13, 13', 13") chaque fois sous un certain espacement (l, l', l") par rapport au corps de luminaire (4), **caractérisé en ce que** l'espacement (l, l', l") d'au moins deux points d'intersection (13, 13', 13") par rapport au corps de luminaire (4) en direction de l'axe médian (12) est différent.

2. Luminaire chirurgical selon la revendication 1, **caractérisé en ce que** des moyens sont prévus pour, indépendamment, faire varier la lumière et mettre en service et hors service les différents moyens d'éclairage (9).

3. Luminaire chirurgical selon l'une des revendications précédentes, **caractérisé en ce que** les moyens d'éclairage (9) sont disposés dans un plan, perpendiculairement à l'axe médian (12).

4. Luminaire chirurgical selon l'une des revendications précédentes, **caractérisé en ce que** plusieurs moyens d'éclairage (9) sont prévus, éclairant, en étant rassemblés en groupes, le point (13, 13', 13") respectif.

5. Luminaire chirurgical selon la revendication 4, **caractérisé en ce que** les moyens d'éclairage (9) d'un groupe sont répartis régulièrement sur le corps de luminaire (4).

6. Luminaire chirurgical selon l'une des revendications précédentes, **caractérisé en ce que** les moyens d'éclairage (9) sont montés sur des surfaces de fixation (15) inclinées.

7. Luminaire chirurgical selon la revendication 6, **caractérisé en ce que** des axes perpendiculaires, sur les surfaces (15) inclinées respectives pour un groupe, se coupent au point (13, 13', 13") sur l'axe médian.

8. Luminaire chirurgical selon l'une des revendications précédentes, **caractérisé en ce que** les moyens d'éclairage (9) sont des LEDs.

9. Luminaire chirurgical selon l'une des revendications précédentes, **caractérisé en ce que** les moyens d'éclairage (9) sont des LEDs avec des réfracteurs.

10. Luminaire chirurgical selon l'une des revendications 1 à 7, **caractérisé en ce que** les moyens d'éclairage (9) sont des LEDs avec des réflecteurs.

11. Luminaire chirurgical selon l'une des revendications 2 à 10, **caractérisé en ce qu'**est prévu un dispositif de commande (7), faisant varier la lumière des moyens d'éclairage (9) ou des groupes de moyens d'éclairage, de manière qu'un groupe d'axes, résultant des différents rayons lumineux (I, I', II, III), présentant la luminosité maximale, ait un point d'intersection avec l'axe médian (12).

12. Luminaire chirurgical selon la revendication 11, **caractérisé en ce que** le luminaire chirurgical (1) comprend un capteur d'espacement pour mesurer un espacement entre le corps de luminaire (4) et le site opératoire et des moyens pour retransmettre l'information d'espacement au dispositif de commande (7).

13. Luminaire chirurgical selon la revendication 11, **caractérisé en ce que** le luminaire chirurgical (1) comprend un capteur de luminosité pour mesurer la luminosité dans le site opératoire et des moyens pour retransmettre l'information de luminosité au dispositif de commande (7).

14. Luminaire chirurgical selon l'une des revendications 12 ou 13, **caractérisé en ce que** le luminaire chirurgical (1) comprend un mécanisme de déclenchement et des moyens pour retransmettre l'infirmation de déclenchement au dispositif de commande (7).
